# EUROPÄISCHE PATENTSCHRIFT

(11) **EP 2 649 994 B1**
(45) Veröffentlichungstag und Bekanntmachung des Hinweises auf die Patenterteilung: **30.08.2017**
(21) Anmeldenummer: 13176138.9
(22) Anmeldetag: 30.01.2012
(51) Int. Cl.: A61K 31/352, A61K 31/385, A61K 31/568, A61K 31/5685, A61K 36/82, A61P 35/00, A61K 45/06

(54) **KOMBINATIONEN VON AROMATASE INHIBITOREN UND ANTIOXIDANZIEN**
COMBINATIONS OF AROMATASE INHIBITORS AND ANTIOXIDANTS
COMBINAISONS D'INHIBITEURS DE L'AROMATASE ET D'ANTIOXYDANTS

(30) Priorität: 31.01.2011 DE 102011003407; 31.01.2011 US 201161437757 P
(43) Veröffentlichungstag der Anmeldung: 16.10.2013
(62) Teilanmeldung aus: 12703265.4
(73) Patentinhaber: LUCOLAS-M.D. Ltd., Birmingham B18 6EW (GB)
(72) Erfinder: Schmidt, Alfred, 68740 Nambsheim (FR)
(74) Vertreter: Prüfer & Partner mbB Patentanwälte · Rechtsanwälte

(56) Entgegenhaltungen:
- WO-A1-97/10808
- WO-A1-2009/108361
- WO-A2-2005/055947
- US-A1- 2002 086 856
- US-A1- 2003 072 821
- US-A1- 2007 072 941
- SATOH K ET AL: "Inhibition of aromatase activity by green tea extract catechins and their endocrinological effects of oral administration in rats", FOOD AND CHEMICAL TOXICOLOGY, Bd. 40, Nr. 7, Juli 2002 (2002-07), Seiten 925-933, XP002671570, ISSN: 0278-6915
- MAGALHÃES ANA CAROLINA ET AL: "Chlorhexidine and green tea extract reduce dentin erosion and abrasion in situ.", JOURNAL OF DENTISTRY DEC 2009 LNKD- PUBMED:19733206, Bd. 37, Nr. 12, Dezember 2009 (2009-12), Seiten 994-998, XP002671571, ISSN: 1879-176X
- PAEPKE S ET AL: "Overview of neoadjuvant endocrine therapy for receptor positive breast cancer in post menopausal women", GEBURTSHILFE UND FRAUENHEILKUNDE 200412 DE, vol. 64, no. 12, December 2004 (2004-12), pages 1290-1298, ISSN: 0016-5751
- DOZIO E ET AL: "The natural antioxidant alpha-lipoic acid induces p27<Kip1>-dependent cell cycle arrest and apoptosis in MCF-7 human breast cancer cells", EUROPEAN JOURNAL OF PHARMACOLOGY, ELSEVIER SCIENCE, NL, vol. 641, no. 1, 1 September 2010 (2010-09-01), pages 29-34, XP027091762, ISSN: 0014-2999 [retrieved on 2010-05-24]
- LEE HYUN SOOK ET AL: "alpha-Lipoic acid reduces matrix metalloproteinase activity in MDA-MB-231 human breast cancer cells", NUTRITION RESEARCH, vol. 30, no. 6, June 2010 (2010-06), pages 403-409, ISSN: 0271-5317
- BARNADAS A ET AL: "Exemestane as primary treatment of oestrogen receptor-positive breast cancer in postmenopausal women: a phase II trial", BRITISH JOURNAL OF CANCER, vol. 100, no. 3, February 2009 (2009-02), pages 442-449, ISSN: 0007-0920(print)
- DEGENHARDT T ET AL: "Individuelle Tumortherapie beim Mammakarzinom", DEUTSCHE ZEITSCHRIFT FÜR ONKOLOGIE, vol. 47, 2015, pages 57-65,

## Beschreibung

Die Erfindung betrifft eine pharmazeutische Medikation zur Behandlung von Östrogenrezeptor-positivem Brustkrebs.

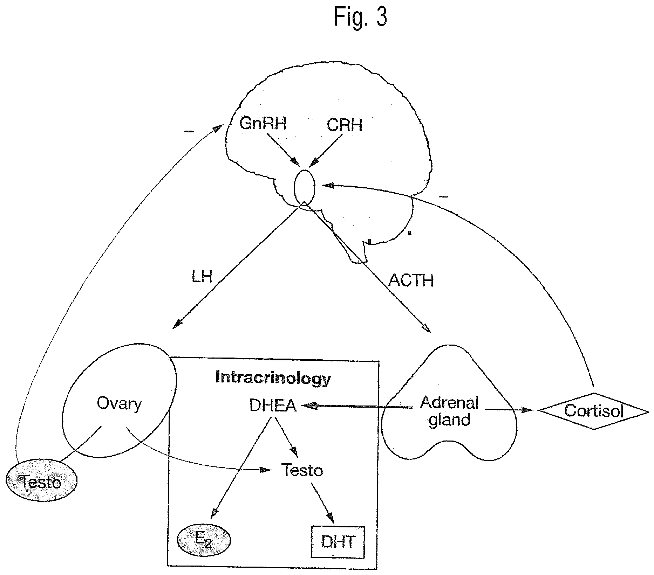

Sexualhormonabhängige Erkrankungen besitzen in der Bevölkerung eine hohe Prävalenz. Zum Beispiel Brustkrebs ist die bei Frauen in Europa am häufigsten diagnostizierte Krebserkrankung. Man rechnet mit 350.000 Neuerkrankungen jährlich, wobei im selben Zeitraum 130.000 Frauen an Brustkrebs sterben (ENCR Cancer Fact Sheets, Vol. 2, Dec. 2002). Somit handelt es sich bei 26,5 % aller Krebserkrankungen um Brustkrebs; 17,5 % aller krebsbedingten Todesfälle werden durch Brustkrebs verursacht.
Die Therapie richtet sich im Einzelfall nach der Kategorisierung des Tumors z.B. anhand des Stadiums, in dem sich die Erkrankung befindet, oder der Tumorbiologie wie z.B. der Expression von Hormonrezeptoren durch die Tumorzellen. Die Expression von Östrogenrezeptoren (ER) oder Progesteronrezeptoren (PR) ermöglicht eine medikamentöse Therapie (z.B. als adjuvante Therapie nach Mastektomie) mit dem Östrogen-Antagonisten Tamoxifen (Early Breast Cancer Trialists' Collaborative Group. N Engl J Med 1988; 319:1681-1692).
Ein weiterer Therapieansatz, der vor allem bei Frauen nach der Menopause verfolgt wird, ist die gezielte Hemmung der Aromatase. Das Enzym Aromatase katalysiert die Umwandlung von Androgenen in Östrogene. Postmenopausal findet diese Reaktion fast vollständig in den peripheren Ziel-Geweben statt, wo das dort synthetisierte Östrogen lokal, d.h. in der Zelle, seine Wirkung entfaltet. Dieser Vorgang kann durch das als 'Intracrinology' bekannte Konzept beschrieben werden (Labrie F., Molecular and Cellular Endocrinology 1991; 78:C113-C118; sowie Labrie F., Nature Clinical Practice Endocrinology & Metabolism 2007, Vol. 3 No. 8; vgl. auch Figur 3). Viele Studien belegen die Wirksamkeit von Aromatase-Inhibitoren in der Brustkrebstherapie bei postmenopausalen Patientinnen mit ER-positiven Tumoren (Übersichtsartikel: Nordman et al., MJA 2005; 183: 24-27; Campos SM, The Oncologist 2004; 9:126-136; Lake DE and Hudis C, Cancer Control 2002; 9: 490-498).
US 2002/0086856 offenbart die Behandlung von Brustkrebs mit einem steroidalen Aromatase-Inhibitor.
Dozio et al. ("The natural antioxidant alpha-lipoic acid induces p27 Kip1-dependent cell cycle arrest and apoptosis in MCF-7 human breast cancer cells", European Journal of Pharmacology, Elsevier Science, NL, Bd. 641, Nr. 1, 1 September 2010, Seiten 29-34) befasst sich mit der Wirkung von alpha-Liponsäure auf die Krebszelllinie MCF-7.
Lee Hyun Sook et al. ("Alpha-Lipopic acid reduces matrix metalloproteinase activity in MDA-MB-231 human breast cancer cells", Nutrition Research, Bd. 30, Nr. 6, Juni 2016, Seiten 403-409) befasst sich mit der Wirkung von alpha-Liponsäure auf Brustkrebszellen.
Trotz der Erfolge, die in der Therapie durch den Einsatz von Aromatase-Inhibitoren erzielt werden konnten, bleibt die Diagnose Brustkrebs lebensbedrohlich und der Bedarf nach einer verbesserten Brustkrebstherapie bestehen.
Neben Brustkrebs gibt es zudem eine Reihe anderer Krankheiten, deren Entstehung und Verlauf sexualhormonabhängig sind und für deren Therapie und Prophylaxe wirksame und sichere Arzneimittel und deren Verwendungen benötigt werden.

Aufgabe der vorliegenden Erfindung ist es, ein wirksames therapeutisches Konzept zur gezielten Behandlung von Östrogenrezeptor-positivem Brustkrebs im Menschen bereitzustellen und ferner Therapien von weiteren sexualhormonabhängigen Erkrankungen zu ermöglichen. Diese Aufgabe wird durch die vorliegende Erfindung wie in Anspruch 1 definiert gelöst. Weiterbildungen sind in den entsprechenden Unteransprüchen festgelegt.

Die vorliegende Erfindung betrifft eine pharmazeutische Medikation, die einen steroidalen Aromatase-Inaktivator und das Antioxidans α-Liponsäure als Wirkstoffe bzw. Wirkkomponenten enthält, zur Verwendung in einer Prophylaxe oder Therapie einer sexualhormon-abhängigen Erkrankung im Menschen, wobei die sexualhormonabhängige Erkrankung Östrogenrezeptor-positiver Brustkrebs ist, und die Medikation topisch appliziert wird, und wobei die Menge des applizierten Antioxidans mindestens 0,5 Gew.-% der Gesamtzusammensetzung beträgt.

Im Sinne der Erfindung wird eine pharmazeutische Medikation zur Therapie oder Prophylaxe von Östrogenrezeptor-positivem Brustkrebs im Menschen verwendet. Dabei wird ein steroidaler Aromatase- Inaktivator mit dem Antioxidans α-Liponsäure kombiniert. In der Kombination verstärken sich die positiven Effekte signifikant und überproportional. Das erfindungsgemäße therapeutische Konzept sieht vor, dass der steroidale Aromatase-Inaktivator durch die zeitgleiche Anwendung des Antioxidans α-Liponsäure eine besondere Wirkung entfaltet. Es wird vermutet, dass das Antioxidans α-Liponsäure in der spezifischen Kombination die prophylaktische oder therapeutische Wirksamkeit des steroidalen Aromatase-Inaktivators unterstützt und dass es zu einem synergistischen Zusammenwirken kommt. Antioxidantien im Sinne der vorliegenden Erfindung agieren im Organismus als sogenannte Radikalfänger, d.h. sie deaktivieren die durch exogene Noxen (z.B. Nikotin oder Alkohol) und endogen induzierten hochreaktiven Radikale.
Bei der Behandlung von Brustkrebs ist die positive Wirkung der Kombination offensichtlich selektiv für Krebszellen, sodass es zu einem verringerten Wachstum und Rückbildung (Apoptose) des Tumors kommt. Es wird vermutet, dass die Selektivität mit der besonderen Situation in Krebszellen zusammenhängt, wo der oxidative Stress im Allgemeinen erhöht ist (Szatrowski TP und Nathan CF, Cancer Res 1991, 51:794-798). Das spezielle Zusammenwirken bzw. der erwähnte synergistische Effekt der Kombination des steroidalen Aromatase-Inaktivators mit dem Antioxidans α-Liponsäure ist umso überraschender, als es Hinweise darauf gibt, dass einige Chemotherapeutika dadurch wirken, dass sie den oxidativen Stress in der Krebszelle erhöhen und diesen somit über eine kritische Grenze anheben und dadurch die Proliferation des Tumors verlangsamen oder aufhalten (Yokomizo A. et al., Cancer Res 1995, 55:4293-4296; Ferlini C. et al., Br J Cancer 1999, 79:257-263; Trachootham et al., Cancer Cell 2006, 10:241-252). Demnach wäre zu erwarten gewesen, dass die Verabreichung eines Antioxidans zusammen mit einem Chemotherapeutikum nicht hilfreich sei, da das Antioxidans den oxidativen Stress reduzieren sollte.
Im Rahmen der vorliegenden Erfindung wurde aber überraschend gefunden, dass die Kombination des Antioxidans α-Liponsäure mit einem steroidalen Aromatase-Inaktivator bei der sexualhormonabhängigen Erkrankungen Östrogenrezeptor-positiver Brustkrebs eine signifikante Wirkung entfaltet, die sich z.B. darin äußert, dass Brusttumoren sich in sehr kurzer Zeit zurückgebildet haben. Dies gilt auch für die gutartigen, flächigen und großvolumigen FettTumore des Morbus Madelung. Es wird angenommen, dass im Fall von gutartigen Tumoren (ähnlich wie bei Krebszellen, siehe oben) die erhöhte Proliferationsrate und der damit verbundene erhöhte (oxidative) Stoffwechsel die Grundlage für die unerwartete Wirksamkeit der erfindungsgemäßen Kombination von einem steroidalen Aromatase-Inaktivator und dem Antioxidans α-Liponsäure darstellt.

Durch die beiden in der Medikation enthaltenen Wirkstoffe bzw. Wirkkomponenten werden unterschiedliche zelluläre Vorgänge und Interaktionen offenbar so beeinflusst, dass der kombinierte Einsatz eines Aromatase-Inhibitors einerseits und eines Antioxidans andererseits zu einem synergistischen Effekt führt. Die erhöhte Wirksamkeit der Wirkstoffe in Kombination ermöglicht die Verwendung der einzelnen Wirkstoffe auch in Dosen, die unter denen der jeweiligen Monotherapien liegen. Dadurch werden unerwünschte Nebenwirkungen z.B. des Aromatase-Inhibitors reduziert oder vermieden.

"Pharmazeutische Medikation" im Rahmen der vorliegenden Erfindung bedeutet, dass die einzelnen Wirkstoffe bzw. Wirkkomponenten getrennt, etwa in separaten Applikationsformen oder Dosiseinheiten, oder als kombinierte Zusammensetzung, etwa in einer gemeinsamen Applikation oder Dosiseinheit oder in Form eines Kits, verabreicht, hergerichtet oder bereitgestellt werden. Im Sinne der erfindungsgemäßen Medikation werden beide Wirkstoffe entsprechend ihren pharmakologischen Eigenschaften so verabreicht bzw. die Medikation so hergerichtet, dass sie ihre physiologischen Effekte gleichzeitig, gleichsinnig und am gleichen Ort entfalten bzw. die Zeitintervalle, während derer die einzelnen Wirkstoffe physiologisch wirksam sind, überlappen. Die überraschende gegenseitige Verstärkung der Wirkstoffe führt zu einer Verbesserung der Wirksamkeit der Medikation in der Therapie und in der Prophylaxe der sexualhormonabhängige Krankheit Östrogenrezeptor-positiver Brustkrebs.

Die Erfindung soll anhand der nachfolgenden Beschreibung von bevorzugten Ausführungsformen und der Figuren im Detail veranschaulicht werden, ohne jedoch das allgemeine Konzept darauf zu beschränken.
Figur 1 zeigt das Ergebnis der Behandlung mit der erfindungsgemäßen Kombination einer Patientin mit einem ER-positiven Tumor in der rechten Brust. Fig. 1A zeigt die Mammographien, Fig. 1B die Auswertung der radiologischen Messung des Tumorvolumens zu Beginn der Behandlung und 29 Tage später.
Figur 2 zeigt das Ergebnis der Behandlung einer anderen Patientin mit einem ER-positiven Tumor in der linken Brust. Fig. 2A zeigt die Mammographien, Fig. 2B die Auswertung die Ergebnisse der radiologischen Messung des Tumorvolumens zu Beginn der Behandlung und 14 Tage später.
Fig. 3 zeigt das bevorzugte Konzept nach dem Prinzip der "Intracrinology" und dem entsprechend bevorzugten Ziel der erfindungsgemäßen Anwendungen.
Aufgrund der besonderen Wirkung der erfindungsgemäßen Verwendung der Medikation kommt deren Anwendung ferner in Betracht bei der Behandlung oder Prophylaxe von hormonabhängig wachsenden Tumoren bei Östrogenrezeptor-positivem Brustkrebs. Wegen des Einsatzes des Antioxidans in der Kombination im Sinne der Erfindung ist die Erfindung besonders nützlich bei der Prophylaxe und Therapie von solchen Erkrankungen oder Zuständen, bei denen sich eine Reduktion der Anzahl der freien Radikale im Organismus, vor allem aber in dem befallenen Organ bzw. Gewebe, positiv auswirkt. Durch den gleichzeitigen, additiven Einsatz eines steroidalen Aromatase-Inaktivators ist die erfindungsgemäße Verwendung besonders wirksam im Falle der sexualhormonabhängigen Erkrankung Östrogenrezeptor-positiver Brustkrebs.

Sexualhormonabhängige Erkrankungen sind sämtliche Krankheiten und pathologische Zustände, die durch die Einwirkung eines oder mehrerer Sexualhormone erzeugt, durch das Ungleichgewicht (Imbalance) der lokalen (extragonadalen) Sexualhormonproduktion in dem betroffenen Organ oder dem betroffenen Gewebe ausgelöst oder in ihrem Verlauf beeinflusst werden. Relevante sexualhormonelle Einflussfaktoren und Ziele, insbesondere in Bezug auf relevante steroidogene Enzyme im peripheren ("intrakinen") Gewebe, die im Zusammenhang mit der Therapie der sexualhormonabhängigen Erkrankung gemäß der Erfindung stehen können, sind aus Fig. 3 ersichtlich, die eine schematische Wiedergabe der Rolle von ovariellen und adrenalen Quellen von Sexhormonen zeigt, die insbesondere für postmenopausalen Frauen typisch sind, hier aber die bevorzugten Anwendungsfälle der vorliegenden Erfindung verdeutlichen soll. Analog der Situation nach der Menopause werden Östrogene und Androgene lokal im peripheren, "intrakrinen" Zielgewebe generiert (vgl. untere Hälfte der Fig. 3). Soweit geringe Mengen Testosteron lokal erzeugt und in den Kreislauf gebracht werden, erfolgt eine inhibitorische Wirkung (-) auf die Sekretion im Gehirn in Bezug auf das Gonadotropin-freisetzende Hormon (GnRH) (vgl. obere Hälfte der Fig. 3). Andererseits setzen adrenale Drüsen Dehydroepiandrosteron (DHEA) frei, wie auch Cortisol, welches seinerseits die Sekretion von des Corticotropinfreisetzenden Hormons (CRH) herabsetzt, das sonst den Spiegel des Adrenocoricotropins (ACTH) stimulieren würde. DHEA wird in den spezifischen peripheren Zielgeweben über das Konzept der 'Intracrinology' in Androgene und/oder Östrogene überführt. Nur geringe Mengen dieser peripher gebildeten Sexhormone gelangen in den systemischen Kreislauf. Somit kann die erfindungsgemäße Anwendung tatsächlich und sehr wirksam im peripheren Zielgewebe, und damit insbesondere lokal und vor allem über topische Applikation, eingreifen.

Die Erfindung betrifft eine pharmazeutische Medikation zur Verwendung gemäß Anspruch 1.

In Kombination mit einer anti-androgenen Therapie, zum Beispiel 5-Alphareduktase-Inhibitoren, LH-RH-Analoga oder Androgenrezeptor-Blockern kann auch die Prostata-Hypertrophie und der Prostatakrebs besonders erfolgreich therapiert werden. Das Wachstum der Prostata ist sowohl in gesunden Individuen als auch in der speziellen Situation des Prostatakarzinoms sexualhormonabhängig. Es ist überdies bekannt, dass die lokale hormonelle Balance, also das Zusammenspiel von Östrogenen und Androgenen im Zielgewebe für den Prostatakrebs von erheblicher Bedeutung ist (Kelloff et al., Cancer Epidemiology, Biomarkers and Prevention 1998;7:65-78).
Der Einsatz eines Antioxidans in Kombination mit einem Aromatase-Inhibitor ist therapeutisch besonders wirksam bei Krankheiten oder Zuständen, deren Entstehung oder Fortschreiten durch Östrogen oder dessen Vorstufen oder Derivate beeinflusst werden. Insbesondere bei solchen Tumoren, die Östrogenrezeptoren exprimieren (ER-positive Tumoren) ist die positive Wirkung von Aromatase-Inhibitoren gezeigt worden (Übersichtsartikel: Labrie F., Nature Clinical Practice 2007;3:584-593). Es wird vermutet, dass beim gleichzeitigen Einsatz eines Aromatase-Inhibitors und eines Antioxidans der Synergismus durch die gezielte Einwirkung auf unterschiedliche physiologische Vorgänge erreicht wird.

Steroidale Aromatase-Inaktivatoren im Sinne der vorliegenden Erfindung sind alle Substanzen, die - unabhängig von ihrer Struktur - durch das gemeinsame Merkmal gekennzeichnet sind, dass sie die Aromatase effektiv inaktivieren (Übersichtsartikel: Santen et al., Endocrine Reviews 2009;30:343-375). Die Fähigkeit einer Substanz, Aromatase zu inaktivieren, kann durch Methoden bestimmt werden, die dem Fachmann bekannt sind. Ein radiometrischer Assay zum Beispiel ermöglicht die Messung der Aromatase-Aktivität in nur einem Schritt durch die Ermittlung der Tritium-Freisetzung von einem tritiummarkierten Substrat (Thompson und Siiteri, Journal of Biological Chemistry 1974;249:5364-5372). Die Gruppe der Aromatase-Inhibitoren ist strukturell heterogen und umfasst sowohl steroidale als auch nicht-steroidale Verbindungen, wobei Vertreter der Gruppe der steroidalen Aromatase-Inaktivatoren für die Medikation im Sinne der Erfindung in Frage kommen. Nicht-steroidale Aromatase-Inhibitoren sind z.B. Anastrazol, Letrozol und Vorozol. Beispiele steroidaler Aromatase-Inaktivatoren gemäß der vorliegenden Erfindung sind 4-Hydroxyandrostendion, Exemestane, 4-Acetoxyandrostendion, 5-α-Androst-3-en-17-on und 3-α,4-α-Epoxy-5-α-androstan-17-on.

Ein Antioxidans gemäß der vorliegenden Erfindung ist ein "Radikalfänger", der freie Radikale einfängt oder deren schädlichen Einfluss in der Zelle stoppt. Gemäß der vorliegenden Erfindung wirkt die Verabreichung eines Antioxidans in Kombination mit einem Aromatase-Inhibitor offensichtlich der Entstehung und dem Wachstum von Krebs entgegen, verlangsamt die Proliferation eines Tumors und/oder bewirkt die Abnahme der Tumormasse, durch Apoptose. Diese Eigenschaften sind sowohl bei der Therapie und Sekundärprophylaxe als auch bei der Primärprophylaxe von Brust - / Prostata - Krebs von entscheidender Bedeutung. Einerseits kann die primäre Entstehung des Tumors verhindert werden, wenn das mutagene Potential durch Reduktion des exogen (z.B. Nikotin- und Alkoholgebrauch)oder endogen (z.B. durch "Abfallprodukte" oder Metabolite des Östrogenmetabolismus) induzierten oxidativen Stress vermindert wird. Andererseits treten während der Progression eines bereits vorhandenen Tumors häufig weitere Mutationen auf, welche die Aggressivität des Tumors erhöhen. Möglicherweise werden die Entstehung solcher weiterer Mutationen und damit die Tumorprogression durch den Einsatz von Antioxidantien verhindert oder verlangsamt. Eine Synergie von Antioxidans und Aromatase-Inhibitor in der Tumortherapie wird vor allem auch dadurch erreicht, dass die freien Radikale, die auch in der Lage sind, Östrogen-Wirkungen zu imitieren (McLachlan, Environmental Health Perspectives 1993; 101:386-387), inaktiviert werden.

Während die Wirkungsweise wie erläutert im Sinne der Erfindung gleichgerichtet und konsistent ist, ist die Gruppe der Antioxidantien strukturell sehr heterogen. Geeignete Substanzen im Sinne der Erfindung werden anhand ihrer Fähigkeit, die Oxidation anderer Moleküle zu verhindern, ausgewählt. Der Fachmann ist in der Lage, ein Antioxidans mittels etablierter und publizierter Methoden zu identifizieren. So kann z.B. die Menge an freien Radikalen durch EPR (*electric paramagnetic resonance*) gemessen werden (Lo Scalzo, EJEAFChe 2010; 9:1360-1371). Dazu werden Substanzen wie z.B. 5,5-Dimethyl-1-pyrrolin-N-oxid (DMPO) oder 1,1-Diphenyl-2-picrylhydrazyl (DPPH) eingesetzt, die eine hohe Affinität zu freien Radikalen haben und mit diesen stabile Verbindungen eingehen, die spektrometrisch messbar sind. Es kommen auch Verfahren zum Einsatz, bei denen die zu messende Substanz chromatographisch (z.B. durch HPLC) gereinigt wird (Yamaguchi et al., Bioscience, Biotechnology, and Biochemistry 1998; 62:1201-1204). Im Sinne der Erfindung werden antioxidative Substanzen verabreicht. Bei den verabreichten Antioxidantien kann es sich um Substanzen unterschiedlicher chemischer Klassen und unterschiedlichen Ursprungs handeln. Dies können auch antioxidative Substanzen sein, die im Organismus vorkommen, deren Menge bzw. Verfügbarkeit jedoch durch die zusätzliche Verabreichung im Rahmen der erfindungsgemäßen Medikation wirksam erhöht werden, und/oder gegebenenfalls gerade am gewünschten Zielort durch geeignete Applikation bereitgestellt werden. Nicht-enzymatische Antioxidantien umfassen insbesondere Flavonoide (z.B. oligomere Proanthocyanidine (OPC), Anthocyane oder Polyphenole wie Quercetin oder Catechin); Vitamine (z.B. Vitamin C, Vitamin E); Carotenoide (z.B. b-Carotin, Lycopen, Lutein); Minerale (z.B. Kupfer, Mangan, Zink, Selen); Hormone (z.B. Melatonin); Steroide (z.B. Kortison); Ubichinone; N-Acetylcystein; α-Liponsäure; ein Grüntee-Extrakt, der eine antioxidativ wirksame Zusammensetzung aus Polyphenolen, optional auch Aminosäuren, Mineralstoffe (Spurenelemente) und Polysaccharide, enthält und vor allem der die speziellen hochantioxidativ wirkenden Polyphenole Epicatechin und Epigallocatechin enthält (z.B. OM24^{®}, erhältlich bei Omnimedica, Schweiz); und Glutathion. Einige Enzyme erfüllen die Funktion von Antioxidantien und werden als enzymatische Antioxidantien bezeichnet, wie z.B. Glutathionperoxidase, Superoxiddismutase und Katalase. Das Antioxidans der pharmazeutischen Medikation der vorliegenden Erfindung ist α-Liponsäure. Im Vergleich zu möglicherweise nur endogen vorkommenden, oder gegebenenfalls nur zufällig oder für andere Zwecke zugegebenen Antioxidantien wird erfindungsgemäß durch Einstellung passender Mengen sichergestellt, dass ein gewünschter therapeutischer Effekt erzielt wird. Dies wird dadurch sichergestellt, dass die bewusst verabreichte Menge an Antioxidans mindestens 0,5 Gew.-% der Gesamtzusammensetzung beträgt, und dass das Antioxidans gezielt an der gewünschten Behandlungsstelle topisch appliziert wird.

Erfindungsgemäß ist das Antioxidans, das zusammen mit einem steroidalen Aromatase-Inaktivator verwendet wird, α-Liponsäure (1,2-Dithiolan-3-pentansäure). α-Liponsäure ist sowohl in den wässrigen als auch in den Fettphasen der Zellen aktiv. Die Substanz wird sowohl gastrointestinal als auch über die Haut hervorragend resorbiert. Dieses eröffnet unterschiedliche Möglichkeiten der Verabreichung. α-Liponsäure wird im Organismus rasch in Dihydroliponsäure umgewandelt. Dihydroliponsäure regeneriert andere, auch endogene Antioxidantien wie Vitamin C und Vitamin E, was zu weiteren verstärkten Effekten bei der Verabreichung von α-Liponsäure führen kann. α-Liponsäure induziert darüber hinaus die Bildung von Glutathion im Gewebe. Zudem regeneriert α-Liponsäure Glutathion aus Glutathiondisulfid.

Im Sinne der Erfindung bezieht sich der Begriff "Behandlung" sowohl auf die Therapie als auch auf die Prophylaxe der sexualhormonabhängigen Erkrankungen Östrogenrezeptor-positiver Brustkrebs.
Erfindungsgemäß wird die Medikation verwendet in einer Therapie oder Prophylaxe von Östrogenrezeptor-positivem Brustkrebs. Der Einsatz eines steroidalen Aromatase-Inaktivators in Kombination mit dem Antioxidans α-Liponsäure ist besonders wirksam im Falle von ER-positiven Tumoren bei postmenopausalen Patientinnen oder bei ER-positiven Tumoren bei männlichen Patienten.

In der erfindungsgemäßen Ausführungsform werden der steroidale Aromatase-Inaktivator und das Antioxidans α-Liponsäure topisch auf die Haut appliziert. Dies kann in Form eines Kombinationspräparates geschehen oder auch durch die zeitgleiche, oder zeitnahe aber zeitlich überlappende, Applikation der beiden Wirkstoffe in Form von zwei separaten Darreichungsformen. Alternativ können die beiden verschiedenen Wirkstoffe auch über unterschiedliche Applikationswege verabreicht werden, sofern sichergestellt ist, dass sowohl der steroidale Aromatase-Inaktivator als auch das Antioxidans α-Liponsäure ihre Wirkung gleichzeitig entfalten bzw. die Dauer der Wirksamkeit der einen Substanz sich mit derjenigen der anderen Substanz überlappt.

Bei der (lokal) topischen Applikation auf die Haut wird ein Wirkstoff oder werden beide Wirkstoffe in geeigneten Trägern (z.B. Cremes, Salben, etc.) auf das zu behandelnde Gewebe bzw. auf das das zu behandelnde Gewebe umgebende Hautareal aufgetragen. Dadurch gelangt der Wirkstoff oder gelangen die Wirkstoffe direkt in die Zielzellen des zu behandelnden Gewebes, d.h. ohne systemischen Umweg und ohne Leberpassage (First Pass Effect). Die Wirkstoffmengen und die Applikationsintervalle werden dabei so gewählt, dass möglichst keine systemischen Plasmaspiegel, sondern nur lokal wirksame Konzentrationen erreicht werden. Dies hat den Vorteil, dass neben der direkten, unmittelbaren Wirkung, Nebenwirkungen der systemischen Wirkstoffgabe reduziert oder vermieden werden. Auch bei längerer Behandlungsdauer oder Langzeittherapie (z.B. in der prophylaktischen Anwendung) können so unerwünschte Nebenwirkungen verhindert oder begrenzt werden.

In einer besonders bevorzugten Ausführungsform für die topische Therapie umfasst die erfindungsgemäße Medikation Hyaluronsäure. Hyaluronsäure verbessert dabei die Resorption des Wirkstoffes bzw. dessen Diffusion durch die Hornhaut, hält jedoch gleichzeitig den Wirkstoff in der Unterhaut und im Fettgewebe (Depot) und ermöglicht so die lokale Wirksamkeit bzw. verhindert die systemische Verteilung des Wirkstoffs über die Blutbahn (Brown and Jones, JEADV 2005; 19:308-318).

Nicht erfindungsgemäß ist eine transdermale Applikation von entsprechenden Dosen über die Haut, um systemisch wirksame Plasmaspiegel zu erreichen. Die Mengenverhältnisse sind hierbei abhängig von den physiko-chemischen Eigenschaften der Wirkstoffe, den Applikationsintervallen und den Formulierungen, die zum Einsatz kommen. Der Fachmann ist in der Lage, die erfindungsgemäße Verwendung an die jeweilige therapeutische oder prophylaktische Situation anzupassen.

In einer der erfindungsgemäßen Ausführungsform werden steroidale Aromatase - Inaktivatoren, wie Exemestane, 4-Hydroxyandrostenedione oder 4-Acetoxyandrostenedione zusammen mit dem Antioxidans α-Liponsäure topisch über die Haut verabreicht. Wegen ihrer lipophilen Eigenschaften werden steroidale Aromatase-Inaktivatoren besonders gut resorbiert und gelangen über das Unterhaut- Bindefettgewebe in das Zielgewebe, z.B. in die weibliche Brust und das Tumorgewebe. Dies gilt insbesondere für die Kombination mit Hyaluronsäure.

Die erfindungsgemäße Medikation wird bevorzugt eingesetzt in der adjuvanten und in der neoadjuvanten Östrogenrezeptor-positiven Brustkrebstherapie.
In einer weiteren Ausführungsform wird die Medikation zur Prophylaxe von Brustkrebs verwendet.
Bei der Primärprophylaxe werden Frauen mit hohem Risiko, an Brustkrebs zu erkranken, behandelt, und zwar bevorzugt mit folgenden Dosierungen, 1 mal täglich 0,5 - 2 g auf jede Brust: 0,05 - 5 Gew.-%, bevorzugt 0,1 - 1 Gew.-%, z.B. 0,5 Gel. -% α-Liponsäure (wobei 0,5 Gew.-% erfindungsgemäß ist) und 0,1 bis 5 Gew.-%, bevorzugt 0,5 - 2 Gew.-%, z.B. 1 Gew.-% Acetoxyandrostenedione, eingearbeitet in eine geeignete Basiscreme, z.B. DAC Basiscreme mit einer Zusammensetzung, in der 100 g DAC enthalten: 0,5 - 10 g, bevorzugt 2 - 7 g, z.B. 4,0 g Glycerolmonostearat; 0,5 - 10 g, bevorzugt 3 - 8 g, z.B. 6,0 g Cetylalkohol; 1 - 15 g, bevorzugt 3 - 10 g, z.B. 7,5 g mittelkettige Triglyceride (z.B. Neutralöl, Miglyol); 10 - 40 g, bevorzugt 20 - 30 g, z.B. 25,5 g Weisses Vaselin; 0,5 - 10 g, bevorzugt 3 - 8 g, z.B. 7,0 g Macrogol-20-glycerolmonosterat; 2 - 25 g, bevorzugt 8 - 15 g, z.B. 10,0 g Propylenglycol; 10 - 80 g, bevorzugt 30 - 60 g, z.B. 40,0 g gereinigtes Wasser).
Bei Frauen, die bereits an Brustkrebs erkrankt sind, wird nach der chirurgischen Therapie eine Sekundärprophylaxe (adjuvante Therapie) durchgeführt. Zudem wird bei Erkrankung einer Brust die kontralaterale Brust prophylaktisch mitbehandelt, z.B. mit folgendem Therapieschema: 2mal täglich (morgens und abends) je 0,5 - 4 g auf jede Brust: 0,05 - 5 Gew.-%, bevorzugt 0,1 - 1 Gel.-%, z.B. 0,5% α-Liponsäure (wobei 0,5 Gew.-% erfindungsgemäß ist) und 0,1 bis 5 Gew.-%, bevorzugt 0,5 - 2,5 Gew.-%, z.B. 1,5% Acetoxy-Androstenedione eingearbeitet in eine Basiscreme (z.B. DAC).

In einer erfindungsgemäßen Ausführungsform wird der steroidale Aromatase-Inaktivator 4-Hydroxy-Androstendion zusammen mit α-Liponsäure angewendet, insbesondere in der neoadjuvanten Therapie des ER-positiven Brustkrebses. Dabei werden die Wirkstoffe gemeinsam oder getrennt voneinander topisch auf die Brust aufgetragen. Die Konzentrationen der α-Liponsäure liegen bevorzugt in einem Bereich von 0,5 Gew.-% bis 2 Gew.-%. Die Konzentrationen des steroidalen Aromatase-Inaktivators liegen bevorzugt in einem Bereich von 1 Gew.-% bis 3 Gew.-%. Ansonsten gilt für das Dosierungsschema bevorzugt das gleiche Therapieregime wie in der Sekundärprophylaxe(adjuvante Therapie). Beispielhafte Dosierungsschemata sind:
1. In der Primärprophylaxe einmal täglich 0,5 - 2 g der topischen Zubereitungsform auf jede Brust und zwar bevorzugt 0,5 Gew.-% α-Liponsäure, 1 Gel.-% Acetoxy-Androstendion.
2. In der Neoadjuvanten Therapie zweimal täglich 2-4 g der topischen Zubereitungsform auf jede Brust in der Konzentration 1 Gew.-% α-Liponsäure und 2 Gew.-% Acetoxy-Androstendion
3. In der Sekundärprophylaxe (Adjuvante Therapie) zweimal täglich 4 g auf jede Brust in der Konzentration 0,5 Gew.-% α -Liponsäure und 1,5 Gew.-% Acetoxy-Androstendion.
Die Dauer der neoadjuvanten Therapie ist abhängig von der Größe des Primärtumors und der Intention des Chirurgen. Da das Ansprechen auf die Therapie bereits nach 2 Wochen sichtbar und messbar ist, kann das therapeutische Ziel relativ exakt definiert werden. Die neoadjuvante Therapie sollte in der Regel jedoch nicht länger als ca. 3 Monate vor der Operation durchgeführt werden. Die Effektivität und Verträglichkeit der erfindungsgemässen Zusammensetzung sind so überzeugend, dass bei älteren Patientinnen (und Patienten) allein mit der topischen Therapie behandelt werden kann. Ein chirurgischer Eingriff ist gerade im hohen Alter ein Risiko.

Beschrieben ist darüber hinaus eine pharmazeutische Zusammensetzung, die einen Aromatase-Inhibitor und speziell die α-Liponsäure umfasst. Bei der Zusammensetzung handelt es sich um ein Kombinationspräparat, durch dessen Verwendung die besonderen technischen Effekte wie oben ausgeführt erzielt werden. Die Wirkung des Aromatase-Inhibitors und die Wirkung der α-Liponsäure sind dabei synergistisch, wie oben bereits beschrieben.

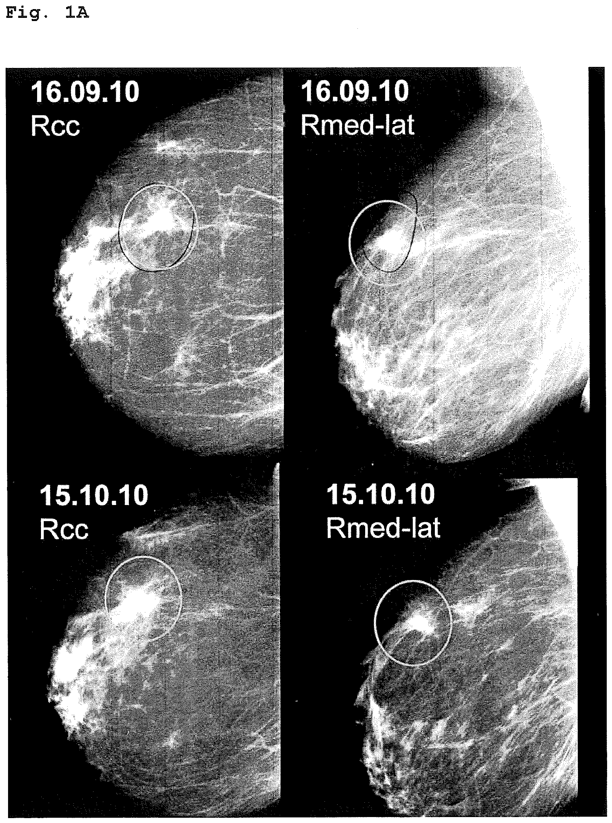

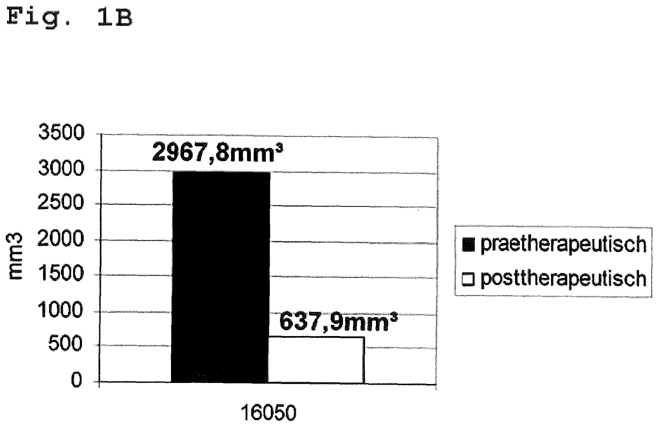

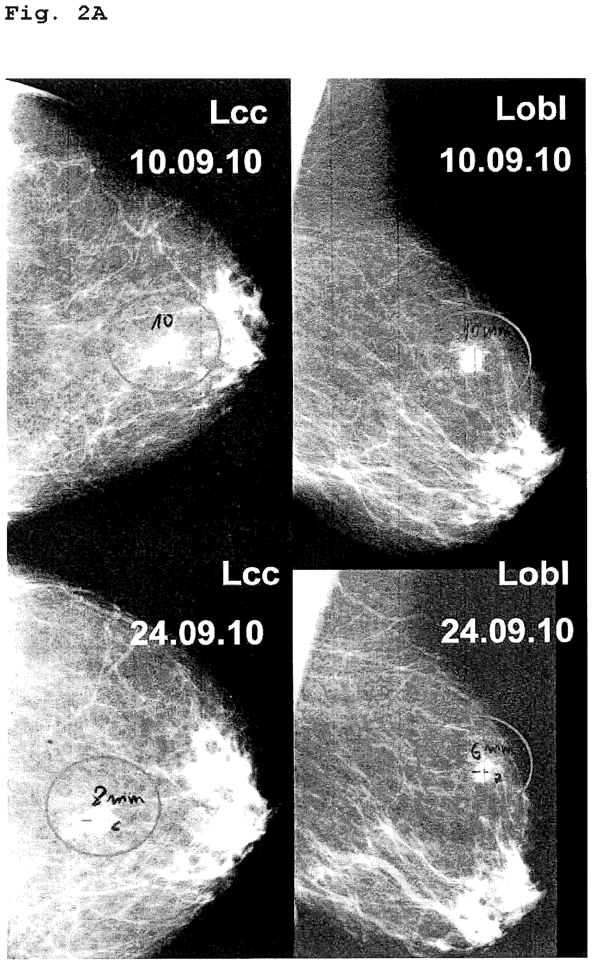

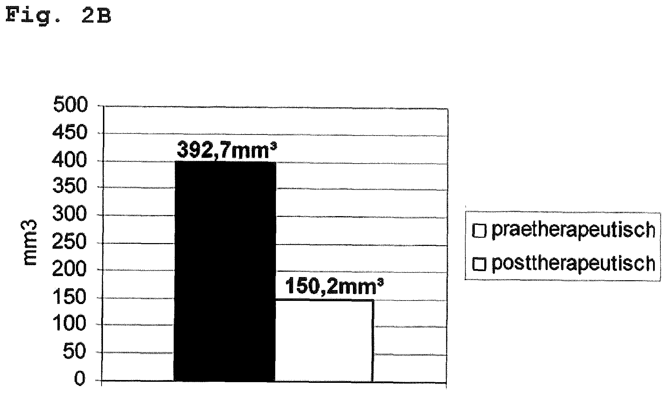

In den Figuren 1 und 2 sind die Ergebnisse der Behandlung bei zwei weiblichen Patienten mit ER-positiven Tumoren der Brust gezeigt. Im Falle einer Patientin wurde nach 29 Behandlungstagen eine Reduktion des Tumorvolumens um 78,5 % gemessen (Fig. 1A, 1B), bei einer anderen Patientin wurde nach 14 Tagen der Behandlung eine Reduktion des Tumorvolumens um 61,8 % erreicht (Fig. 2A, 2B).

Im Sinne der vorliegenden Erfindung wird eine pharmazeutische Medikation, die einen steroidalen Aromatase-Inaktivator und das Antioxidans α-Liponsäure umfasst, zur Behandlung der sexualhormonabhängigen Erkrankungen Östrogenrezeptor-positiver Brustkrebs verwendet. Darüber hinaus kann die Medikation ferner zur Behandlung der Mastopathie, der Mastodynie und probatorisch der Mastalgie verwendet werden.
Die Mastopathie ist eine gutartige Veränderung des Drüsenkörpers der Brust. Sie tritt bei mindestens jeder zweiten Frau auf und ist somit die häufigste Brustdrüsenerkrankung der Frau. Die betroffenen Frauen sind meist 35 bis 50 Jahre alt; eine Mastopathie bei Frauen, die jünger als 25 Jahre oder schon in den Wechseljahren sind, ist sehr selten.

Je nachdem, wie das Drüsengewebe der Brust verändert ist, lassen sich verschiedene Formen von Mastopathie unterscheiden:
- Fibröse Mastopathie (Mastopathia fibrosa): Bei dieser Form ersetzt glasartiges Bindegewebe zunehmend die feine Gewebsschicht, die die Drüsengänge nach innen auskleidet (Epithel).
- Fibrozystische Mastopathie (Mastopathia fibrosa cystica): Diese Form ist gekennzeichnet durch eine Vermehrung des Bindegewebes und Erweiterung der Drüsengänge.
- Fibroadenomatöse Mathopathie (Mastopathia fibroadenomatosa): Typisch für diese Form ist eine geschwulstartige Vermehrung von Drüsengewebszellen (sog. adenomatöse Hyperplasie) in den Drüsengängen, die mit Blut, Eiter oder Sekret gefüllt sein können.

Bei der Mastopathie erfolgt außerdem eine Einteilung in drei Gruppen, deren Definition sich nach dem Schweregrad der Veränderung des Brustdrüsengewebes richtet. Durch die Bestimmung des Schweregrads lässt sich das Risiko der betroffenen Frauen für Brustkrebs einschätzen.
Bei der Mastopathie zielt die Therapie in erster Linie darauf ab, die durch die Veränderungen des Brustdrüsengewebes entstehenden Symptome zu lindern. Da ein Ungleichgewicht im Hormonhaushalt für die Mastopathie verantwortlich ist, besteht die bisherige Behandlung vor allem darin, den Östrogenüberschuss durch Gestagen auszugleichen.
Gegen die bei einer Mastopathie auftretenden Spannungszustände und die zystischen Veränderungen der Brust können möglicherweise Prolaktin-Hemmer helfen. Wenn die Mastopathie schwere Symptome verursacht, kann zur Therapie auch zusätzlich Danazol verabreicht werden, das die Freisetzung von Östrogen hemmt.

In sehr seltenen Fällen von Mastopathie kann es auch notwendig sein, die Brust zu entfernen. Diese Therapie kommt besonders bei Frauen infrage, die sehr große Angst vor Brustkrebs haben und bei denen mehrere Risikofaktoren für eine Entartung der Mastopathie zusammentreffen. Dazu zählen eine mehrfach durch Gewebeentnahmen gesicherte Mastopathie Grad III, eine schlechte mammographische Überwachbarkeit der Mastopathie und Fälle von Brustkrebs in der Familie. Diese Risikofaktoren sind vor allem dann von Bedeutung, wenn sich die Mastopathie vor dem 40. Lebensjahr ausbildet. Beispiel 6 zeigt das "Verschwinden" einer fibrozystischen Mastopathie bei einer 49. jährigen Frau nach ca. 12 monatiger Behandlung mit der erfindungsgemäßen Medikation.
Die Mastodynie und die Mastalgie gehören zu den sogenannten funktionellen Störungen der Brust. Beide Begriffe werden in der Fachsprache häufig zur Unterscheidung zyklusabhängiger (ca. 80%) und zyklusunabhängiger (ca.20%) Schmerzen verwendet. Dabei steht die Mastodynie für zyklus- und hormon(Östrogen)abhängige Schmerzen, die Mastalgie für zyklusunabhängige Schmerzen, die nur selten hormonabhängig sind.
Bei der Mastodynie sind die Symptome zyklusabhängig und treten in der Zeit vor und während der Menstruation auf. Schmerzen, Überempfindlichkeit für Berührungsreize, Ziehen, Schwellungen mit Spannungsgefühlen, Schweregefühl in und an beiden Brüsten, tastbare Verhärtungen (prämenstruell) sind mit Abstand die häufigsten Brustbeschwerden, oft schon 2 Wochen vor der Menstruation beginnend. Diese Beschwerden begleiten viele Frauen in ihrem gesamten prämenopausalen Lebensabschnitt. Oft erleiden die Frauen ab dem 30. Lebensjahr eine merkliche Zunahme dieser Beschwerden.
Ca. 80% aller Patientinnen mit Brustschmerzen leiden unter zyktischen Brustschmerzen. 30% der Patientinnen mit Mastodynie haben eine erhebliche Beeinträchtigung der Lebensqualität und dieses auch aus Angst und Stress, die aus dem Wissen resultieren, mit den wiederkehrenden Schmerzen Monat für Monat leben zu müssen.

Neben allgemeinen Massnahmen, wie gut angepasster BH, fettarme Ernährung und die Empfehlung, viel pflanzliche Nahrung zu sich zu nehmen, ist eine Therapie zumindest bei den 30% der Patientinnen mit schweren Symptomen mit pharmazeutischen Substanzen indiziert. Neben einer lokalen Applikation von Progesteron- (und Diclofenac-) Gel wird dabei eine systemische Gabe des Östrogenantagonisten Tamoxifen, des Testosteronderivats Danazol, die Gabe von Bromocriptin (einem Dopamin Agonisten / Prolaktin - Sekretions - Hemmer) verordnet. Die Nebenwirkungen dieser Therapieformen sind zum Teil erheblich.

Ein weiteres nicht-erfindungsgemäßes Einsatzgebiet der pharmazeutischen Medikation sind sexualhormonabhängig wachsende gutartige Tumore, die auf einer lokalen Zunahme von Fettgewebe beruhen. Östrogen fördert die Zunahme von Fettgewebe. Das neu entstandene Fett steigert die körpereigene Produktion von Östrogen und freien Radikalen über die Fettzellen. Es wird vermutet, dass in den Fettzellen die Aktivität des Enzyms Aromatase zunimmt, folglich steigert sich die Umwandlungsaktivität von Testosteron in Östrogen und die Entstehung freier Radikale. Zusätzlich nimmt auch die Anzahl der Rezeptoren, an denen Östrogen und freie Radikale wirksam sein können, in den Zellen zu. Dieses führt wiederum zu einer verstärkten Wirkung der Östrogene und freien Radikale. Eine fettgewebs - immanente autonome Situation "circulus vitiosus", der die pharmazeutische Medikation effektiv entgegenwirkt.
So konnte erstmals gezeigt werden, dass das Lipom (gutartiger Tumor der Fettgewebszellen) und die Lipomatosen sexualhormonabhängig wachsende gutartige Fettgeschwülste sind. Bei den Lipomatosen handelt es sich um eine diffuse Zunahme des Fettgewebes
- meistens ausgehend vom Unterhautfettgewebe - an bestimmten Stellen des Körpers, so können der Hals, der obere Stamm (z.B. Rücken) oder die Hüften, aber auch alles in Kombination oder gemeinsam betroffen sein. Als Synonyme der Lipomatose werden u.a. verwendet:
   Symmetrische Adenolipomatose;
   Lipomatosis symmetrica;
   diffuse symmetrische Lipomatose;
   generalisierte symmetrische Lipomatose;
   multiple symmetrische Lipomatose;
   umschriebene symmetrische Lipomatose;
   Lipomatosis simplex indolens;
   Launois-Bensaude-Syndrom;
   Madelung-Krankheit.
Sowohl für die Lipome als auch für alle Formen der Lipomatose gab es bisher keine kausale Therapie. Operative Reduktion oder Fettabsaugung sind prinzipiell möglich, aber nicht ungefährlich und mit einer hohen Rezidivrate verbunden. Mit der hierein beschriebenen pharmazeutischen Medikation konnte erstmals gezeigt werden, dass das, wie oben beschrieben, sexualhormonabhängig wachsende Fettgewebe mit der Kombination eines Radikalfängers (z.B. 0,5% α-Liponsäure) mit einem Aromatase-Inhibitor (z.B. 1,5% 4-Acetoxyandrostendion) in Kombination mit dem Freisetzungssystem der Hyaluronsäure (z.B. 0,2%) nicht nur das Wachstum der Fetttumoren gebremst wird, sondern die Fetttumore zum "Einschmelzen" gebracht werden. Beispiel 8 zeigt diesen Effekt bei einer 56-jährigen Patientin mit schwerster Madelungscher Erkrankung. Beispiel 9 das Einschrumpfen eines ca. 12 cm großen, eiförmigen Lipoms oberhalb des linken Schultergelenks bei einem 71-jährigen Patienten.

Auch die folgenden, nicht zur Erfindung gehörigen Punkte sind hierin beschrieben:
1. Pharmazeutische Medikation, die einen Aromatase-Inhibitor und ein vom Aromatase-Inhibitor verschiedenes Antioxidans als Wirkstoffe umfasst, zur Verwendung in einer Prophylaxe oder Therapie von hormonabhängigen Erkrankungen.
2. Pharmazeutische Medikation, die einen Aromatase-Inhibitor und ein vom Aromatase-Inhibitor verschiedenes Antioxidans als Wirkstoffe umfasst, zur Verwendung in einer Prophylaxe oder Therapie von Brustkrebs.
3. Pharmazeutische Medikation zur Verwendung gemäß Punkt 2, wobei die Medikation zur adjuvanten oder neoadjuvanten Therapie von Brustkrebs verwendet wird.
4. Pharmazeutische Medikation zur Verwendung gemäß Punkt 2, wobei die Medikation zur Primär- oder Sekündärprophylaxe von Brustkrebs verwendet wird.
5. Pharmazeutische Medikation, die einen Aromatase-Inhibitor, einen 5-Alphareduktase-Hemmer und ein vom Aromatase-Inhibitor verschiedenes Antioxidans als Wirkstoffe umfasst, zur Verwendung in einer Prophylaxe oder Therapie von Prostatakrebs und Prostata-Hypertrophie.
6. Pharmazeutische Medikation zur Verwendung gemäß Punkt 5, wobei die Medikation mit einem oder mehreren anti-androgenen Wirkstoffen, insbesondere 5-a-Reduktase-Inhibitoren oder Androgenrezeptor-Blockern, kombiniert wird.
7. Pharmazeutische Medikation, die einen Aromatase-Inhibitor und ein vom Aromatase-Inhibitor verschiedenes Antioxidans als Wirkstoffe enthält, zur Verwendung in einer Prophylaxe oder Therapie von Mastopathien.
8. Pharmazeutische Medikation, die einen Aromatase-Inhibitor und ein vom Aromatase-Inhibitor verschiedenes Antioxidans als Wirkstoffe umfasst, zur Verwendung in einer Prophylaxe oder Therapie von Mastodynie oder Mastalgie.
9. Pharmazeutische Medikation, die einen Aromatase-Inhibitor und ein vom Aromatase-Inhibitor verschiedenes Antioxidans als Wirkstoffe umfasst, zur Verwendung in einer Prophylaxe oder Therapie von gutartigen Tumoren.
10. Pharmazeutische Medikation zur Verwendung gemäß Punkt 9, wobei die gutartigen Tumoren Lipomatosen sind.
11. Pharmazeutische Medikation zur Verwendung gemäß Punkt 9 oder 10, wobei die gutartigen Tumoren Lipomatosen vom Typ eines Morbus Madelung sind.
12. Pharmazeutische Medikation zur Verwendung gemäß irgendeinem der vorangehenden Punkte, wobei der steroidale Aromatase-Inaktivator ausgewählt ist aus der Gruppe bestehend aus 4-Hydroxyandrostendion, Exemestane, 4-Acetoxyandrostendion, 5-α-Androst-3-en-17-on und 3-α,4-α-Epoxy-5-α-androstan-17-on.
13. Pharmazeutische Medikation zur Verwendung gemäß irgendeinem der vorangehenden Punkte, wobei das Antioxidans ausgewählt ist aus der Gruppe bestehend aus Flavonoiden; Vitaminen; Carotenoiden; Mineralen; Hormonen; Steroiden; Ubichinonen; N-Acetylcystein; α-Liponsäure; Grüntee-Extrakt enthaltend Polyphenole, insbesondere OM24^{®}; Glutathion; Glutathionperoxidase; Superoxiddismutase und Katalase.
14. Pharmazeutische Medikation zur Verwendung gemäß irgendeinem der vorangehenden Punkte, wobei die Medikation als Wirkstoffe 4-Hydroxyandrostendion und α-Liponsäure umfasst.
15. Pharmazeutische Medikation zur Verwendung gemäß irgendeinem der vorangehenden Punkt, wobei die Medikation als Wirkstoffe Exemestane und Grüntee-Extrakt enthaltend Polyphenole, insbesondere OM24^{®}, umfasst.
16. Pharmazeutische Medikation zur Verwendung von hormonabhängigen Erkrankungen, wobei der Wirkeingriff allein in einem peripheren, intrakrinen Zielgewebe erfolgt.
17. Pharmazeutische Medikation zur Verwendung gemäß irgendeinem der vorangehenden Punkte, wobei die Medikation lokal, insbesondere topisch appliziert wird.
18. Pharmazeutische Zusammensetzung, die
   - einen Aromatase-Inhibitor und
   - α-Liponsäure und/oder Grüntee-Extrakt enthaltend Polyphenole, insbesondere OM24^{®}, enthält.
19. Zusammensetzung gemäß Punkt 18, wobei der Aromatase-Inaktivator ausgewählt ist aus der Gruppe bestehend aus 4-Hydroxyandrostendion, Exemestane, 4-Acetoxyandrostendion, 5-α-Androst-3-en-17-on und 3-α,4-α-Epoxy-5-α-androstan-17-on.
20. Zusammensetzung gemäß Punkt 18 oder 19, die Hyaluronsäure als Hilfsstoff umfasst.

Die vorliegende Erfindung wird durch die nachfolgenden Beispiele weiter veranschaulicht.

Beispiele 1 - 5 veranschaulichen die erfindungsgemäße Verwendung der Medikation in der Brustkrebstherapie.

### Beispiel 1:

Bei einer 59-jährigen Patientin wurde ein ER-positiver Tumor in der rechten Brust diagnostiziert. Vor dem Beginn der Behandlung wurde der Tumor mammographisch vermessen (Fig. 1A). in den folgenden 29 Tagen wurden α-Liponsäure 0,5% und 4-Hydroxyandrostendione 1,5% (eingearbeitet in die Basiscreme DAC) 2mal täglich, jeweils 2g topisch auf jede Brust aufgetragen. Anschließend wurde der Tumor erneut vermessen (Fig. 1A) und das Tumorvolumen vor und nach der Behandlung in einem Diagramm graphisch dargestellt (Fig. 1B). Das Tumorvolumen hat sich im Verlauf der 29-tägigen Behandlung um 78,5 % reduziert.

### Beispiel 2:

Eine 69-jährige Patientin wurde mit einem ER-positiven Tumor in der linken Brust diagnostiziert. Es erfolgte eine 14-tägige Behandlung der Brüste durch topische Administration von α-Liponsäure 0,5% und 4-Hydroxyandrostendione 1,5% (eingearbeitet in die Basiscreme DAC) 2mal täglich, jeweils 2g. Das Tumorvolumen vor und nach der Behandlung wurde wie unter Beispiel 1 beschrieben ermittelt (Fig. 2A). Das Volumen des Tumors war nach der Behandlung um 61,8 % reduziert.

### Beispiel 3:

Bei einer 53-jährigen Patientin wurde ein ER-positiver Tumor in der rechten Brust diagnostiziert. Vor dem Beginn der Behandlung wurde der Tumor mammographisch vermessen.
In den folgenden 24 Tagen wurden α-Liponsäure 0,5% und 4-Hydroxyandrostendione 1,5% (eingearbeitet in die Basiscreme DAC) 2mal täglich, jeweils 2g topisch auf jede Brust aufgetragen. Anschließend wurde der Tumor erneut vermessen und das Tumorvolumen verglichen. Das Tumorvolumen hat sich im Verlauf der 24-tägigen Behandlung um 76,0 % reduziert.

### Beispiel 4:

Bei einer 62-jährigen Patientin wurde ein ER-positiver Tumor in der linken Brust diagnostiziert. Es erfolgte eine Behandlung in Form einer topischen Applikation mit der erfindungsgemässen Zusammensetzung. In den folgenden 14 Tagen wurden α-Liponsäure 0,5% und 4-Hydroxyandrostendione 1,5% (eingearbeitet in die Basiscreme DAC) 2mal täglich, jeweils 2g topisch auf jede Brust, aufgetragen. Das Tumorvolumen vor und nach der Behandlung wurde wie unter Beispiel 1 beschrieben ermittelt. Das Volumen des Tumors war nach der Behandlung um 64,5 % reduziert.

### Beispiel 5:

Eine 47 -jährige Patientin litt an einem ER-positiven Tumor in der rechten Brust. Für 28 Tage wurde, wie unter Beispiel 1 und 2 beschrieben, behandelt und das Tumorvolumen jeweils zu Beginn und zum Ende der Behandlung ermittelt. Es zeigte sich dabei eine Reduktion um 60,5 %.

### Beispiel 6 (fällt nicht unter die Ansprüche):

Erfindungsgemäße Verwendung der Medikation bei Mastopathie.

S.H.C., weiblich, 49 Jahre.

Für die Anwendung wurde folgende Kombination ausgewählt:
Als wirksame Bestandteile
   OM 24 0,4%
   Exemestane 1.0%,
Als Hilfsmittel (Resorptions- und Distributionshilfe) Hyaluronsäure 0.3%.

### Anamnese und Therapie:

Bei der Patientin wurde eine fibrozystische Mastopathie Grad II festgestellt. Auf Wunsch der Patientin wurde zur Abwendung des Brustkrebsrisikos ein Therapieversuch mit der oben genannten Kombination, einmal täglich 2g auf beide Brüste, durchgeführt. Nach 11 monatiger Applikation hat sich die Mastopathie (Mastpoathia fibrosa cystica) vollständig zurück gebildet. Das Brustgewebe zeigt eine geordnete Struktur ohne jeglichen Mikrokalk.

### Beispiele 7 und 8 (fallen nicht unter die Ansprüche):

Erfindungsgemäße Verwendung der Medikation bei Mastodynie.

Für die Anwendung wurde folgende Kombination ausgewählt:
Als wirksame Bestandteile:
   α-Liponsäure 0.5%,
   4 - Acetoxy-Androstenedione 1.0%,
Hyaluronsäure 0.2% (lediglich als Resorptions- und Distributionshilfe).

### Beispiel 7

I.H., weiblich, 21. Jahre.

Anamnese: seit dem 14. Lebensjahr monatlich auftretende Menstruationsschmerzen, die während der Vorbereitung auf das Abitur (mit 18.Jahren) so stark wurden, dass ihr Gynäkologe eine Mammographie durchführte, die jedoch lediglich den Befund einer geringgradigen Mastopathie in den oberen Quadranten zeigte. Bisherige Verordnung: Voltaren-Gel mehrfach tägliche Applikation "nach Bedarf".
Aktueller Befund: 3 - 5 Tage vor jeder Menstruation mit starkem Spannungsempfinden auftretende Schmerzen in beiden Brüsten von den oberen Quadranten scheinbar gebündelt in die Brustwarzen einschießend. Unter Voltaren-Gel weiter bestehend, während der gesamten Menstruationsperiode, danach langsam abnehmende Schmerzen, Spannungsgefühle und Ziehen bis etwa zum 12 Tag nach Eintritt der Menstruation.

Individueller Anwendungsversuch mit der hierin beschriebenen Medikation:
4g einmal täglich und zwar 2g auf jede Brust aufgetragen und vorsichtig einmassiert. Der individuelle Anwendungsversuch begann ca. 2 Wochen vor Einsetzen der Menstruation.

### Ergebnis des individuellen Anwendungsversuches:

Die nächste Menstruation verlief mit deutlich gemindertem Spannungsschmerz in der Brust. Gesamtsymptomatik: weniger einschränkend und schneller nachlassend. Bereits 5 Tage nach Menstruationsbeginn war die Patientin schmerzfrei. Weiterführung des Anwendungsversuches mit täglicher Applikation.

Ergebnis nach der zweiten Folgemenstruation: Schmerzfreiheit Fortführung des Versuches mit der erfindungsgemäßen Medikation ohne jegliche unerwünschte Begleiterscheinung.

### Beispiel 8

S. P., weiblich, 32.Jahre, verheiratet;
Anamnese: prämenstruelles Syndrom mit starker Schmerzsymptomatik in beiden Brüsten seit der Pubertät. Ab dem 28. Lebensjahr dynamische Zunahme der Symptomatik mit äusserst starker Berührungsempfindlichkeit, starkes Schwellungs- Spannungsgefühl mit diffus in den Brustkorb und die Arme ausstrahlender Schmerzsymptomatik, die bis zum Eintritt der Menstruation schier unerträglich wird. Seit 3 Jahren 2 bis 4 Tage pro Monat durch diese Beschwerden arbeitsunfähig. 14 Tage pro Monat wegen der pathologisch taktilen Überempfindlichkeit keine intimen Berührungen möglich. Abklingen der Symptomatik erst 7-10 Tage nach Menstruationseintritt. Mammographie vor 6 Monaten ohne Befund. Zur Dokumentation des prämenstruellen Syndroms, führen eines differenzierten Schmerztagebuches seit einem Jahr.
Bisherige Verordnung: über Jahre Testosteron-Gel und / oder Voltaren-Gel praktisch ohne Effekt.
Das gleiche gilt für die vorübergehenden Therapie-Versuche mit Bromocriptin oder Danazol. Lediglich die Gabe von Tamoxifen 20mg führte zu einer erheblichen Linderung der Symptomatik, verursachte aber ebenso erhebliche Nebenwirkungen, vor allem im Bereich des Herz-Kreislaufes (unkontrollierter Blutdruckanstieg). Dieser führte zu einem Absetzen der Therapie und bereits nach 2 Menstruationszyklen zeigte die Symptomatik wieder ihren ursprünglichen Ausprägungsgrad.

Individueller Anwendungsversuch mit der hierin beschriebenen Medikation: 4g, 2 x täglich (morgens und abends) und zwar jeweils 2g auf jede Brust aufgetragen und vorsichtig einmassiert. Der Anwendungsversuch begann ca. 2 Wochen vor Einsetzen der Menstruation.

### Ergebnis des Anwendungsversuches:

Die nächste Menstruation verlief mit deutlich gemindertem Spannungsschmerz in der Brust. Gesamtsymptomatik verläuft weniger einschränkend und lässt schneller nach. Bereits 5 Tage nach Menstruationsbeginn war die Patientin schmerzfrei.

### Weiterführung des Versuches mit täglicher Applikation:

Folgeperiode: deutlich geringere Schwellungs- und Spannungs-Symptomatik, merkbar geringere Schmerz-Symptomatik, hauptsächlich in den Brüsten, wenig ausstrahlend, keine Arbeitsunfähigkeit.

### Fortführung des Anwendungsversuches:

3. Menstruation: weitere Abnahme der Schwellungs- und Spannungsgefühle, geringere Schmerzen, nur noch in den Brüsten, erheblich weniger Berührungsempfindlichkeit, keine Arbeitsunfähigkeit.

Weitere Anwendungsperioden: kaum noch Schwellungs- und Spannungsgefühle, kaum noch Berührungsempfindlichkeit, praktisch Schmerzfrei, keine Mastodynie bedingte Arbeitsunfähigkeit.Während der gesamten Anwendungsperiode keinerlei unerwünschte Begleiterscheinungen.

### Beispiele 9 und 10 (fallen nicht unter die Ansprüche):

Verwendung der Medikation bei gutartigen Tumoren (Fettgeschwülsten).

Für diese Anwendung wurde folgende Kombination ausgewählt: als wirksame Bestandteile Alpha-Liponsäure 1,0%, 4-Hydroxiandrostendion 1,5%, Hyaluronsäure 0,2% (als lokales Freisetzungssystem)eingearbeitet in eine Basiscreme (DAC).

### Beispiel 9

Patientin: E.M.S., 56 Jahre: die Patientin leidet seit annähernd 15 Jahren unter einer sehr seltenen, extremen Fettverteilungsstörung (Inzidenz: 1:25.000 bei Männern und 1:300.000 bei Frauen) durch einen Morbus Madelung, der zum Einen zu einer grotesken Umverteilung von Fettgewebe mit weitgehendem Schwund der Brüste und des Gesäßes und einer Oberkörper, stammbetonten, symmetrischen Lipomatose geführt hat und zum Anderen zu Bewegungseinschränkungen durch die störenden Fetttumore. Zudem hat Frau S. erhebliche Schmerzen in den Fettgeschwülsten am Nacken, am Rücken, am Hals, an der seitlichen Thoraxwand, den Flanken über der Schulter und an den Oberarmen. Dieses entspricht dem typischen Bild eines Morbus Madelung mit Lipomatosis dolorosa. Die Patientin wurde in den Jahren 1995 bis heute mehrfach in Zyklen operiert. Für die letzte Operationsserie (Rücken und Hals, jeweils halbseitig in getrennten Operationen, Oberarme und Flanken ebenso) im Jahre 2008 waren insgesamt 6 OP-Termine notwendig. Bis Dezember 2010: Rezidiv in allen Lokalisationen; noch mehr gesteigertes Wachstum der großflächigen Fetttumore.
Die groteske Umbildung der figürlichen Optik und die erhebliche Schmerzsymptomatik verbunden mit der Problematik, eine adäquate Garderobe zu tragen, haben zu einer schwer ertragbaren Einschränkung der Lebensqualität geführt. Erschwerend kommt hinzu, dass die Patientin seit Jahren aus Schamgefühl und wegen der Schmerzen jeglichen Intimkontakt mit ihrem Partner meidet.
Der Zustand der Patientin war vor Beginn des Therapieversuches mit dem erfindungsgemäßen Medikament schlechter als zu Beginn des letzten OP-Zyklus im Jahre 2008.
Naturgemäß fürchtet die Patientin ein erneutes operatives Vorgehen, da natürlich auch dann wieder mit multiplen Rezidiven zu rechnen ist.
Beginn des individuellen Heilversuches, am 14.12.2010; 2mal täglich wird die hierin beschriebene pharmazeutische Zusammensetzung in alle Fettgeschwülste einmassiert. Wöchentlich werden folgende Areale gemessen: Nacken, Brust, Oberarme links und rechts, Hüfte und Hals.
Ergebnis: es konnte nicht nur das überschießende Wachstum gestoppt werden, sondern es wurden folgende Ergebnisse erzielt: innerhalb von einer bisher 6-wöchigen Therapie nahmen die Umfänge wie folgt ab:

| | | | |
|---|---|---|---|
| Nacken von | 36cm | auf | 31,5cm, |
| Brust von | 116cm | auf | 111,5cm, |
| Arm links von | 42cm | auf | 34cm, |
| Arm rechts von | 36cm | auf | 33cm, |
| Hüfte von | 104cm | auf | 101cm, |
| Halsumfang von | 42cm | auf | 38,5cm (siehe Tabelle 1). |

**Tabelle 1: Ergebnisse der Messungen in cm (Beispiel 8)**

| **DATUM** | **NACKEN** | **BRUST** | **ARM li** | **ARM re** | **HUEFTE** | **Halsumfgang** |
|---|---|---|---|---|---|---|
| 14.12.2010 | 36 | 116 | 42 | | | 42 |
| 21.12.2010 | 35 | 114 | 37 | | 103 | 41 |
| 28.12.2010 | 34 | 114 | 36 | 33 | 102 | 40 |
| 05.01.2011 | 33 | 114 | 35 | 33 | 101 | 40 |
| 12.01.2011 | 32 | 112 | 34 | 33 | 101 | 39 |
| 19.01.2011 | 32 | 112 | 34 | 33 | 101 | 39 |
| 26.01.2011 | 31,5 | 111,5 | 34 | 33 | 101 | 38,5 |

Durchschnittlich verbraucht die Patientin etwa 100g der hierin beschriebenen pharmazeutischen Zusammensetzung pro Woche, das bedeutet bei zweimaliger Applikation pro Tag, eine Wirkstoffkonzentration von 140 mg α-Liponsäure und ca.
214 mg 4-Hydroxyandrostendion.
Unter der jetzigen Behandlung keinerlei unerwünschte Wirkungen; der Heilversuch wird fortgesetzt.

### Beispiel 10

Bei dem 71-jährigen Patienten E.H.S. entwickelte sich über dem linken Schultergelenk innerhalb eines Jahres ein Lipom, welches zunächst die Größe eines Taubeneis hatte. Bis zum Beginn des Heilversuches mit der erfindungsgemäßen Zusammensetzung vergrößerte sich das Lipom zu einem eiförmigen Gebilde von ca. 12cm Längsschnitt, 8cm Querschnitt und 2,5cm Höhe. Neben dem kosmetischen Aspekt beeinflusst das Lipom unter Schmerzentwicklung die Höhenbewegung des linken Arms und Schultergelenks, weil das Lipom offenbar durch Druck auf die Supraspinalsehne im Schultergelenk bei der Bewegung einen erheblichen Schmerz auslöst. Der Patient wollte sich einer chirurgischen Behandlung unterziehen. Der Patient hat sich nun aber im Oktober 2010 entschieden, die hierin beschriebene pharmazeutische Zusammensetzung zu benutzen. Anwendung: 2mal täglich ca. 2g in den gesamten Lipombereich einmassieren.

### Ergebnis: nach 10 - wöchiger Behandlung:

Das Lipom schrumpfte

| | | | |
|---|---|---|---|
| im Längsschnitt von | 12cm | auf | 7cm, |
| im Querschnitt von | 8cm | auf | 4cm, |
| in der Höhe von | 2,5cm | auf | 1cm. |

Der Druck - und Bewegungsschmerz sind erheblich gemindert, so dass der linke Arm wieder annähernd frei beweglich ist.
Da die Anwendung der erfindungsgemäßen Zusammensetzung nicht nur hocheffektiv ist, sondern daneben auch ein angenehmes Hautgefühl vermittelt, wird der Heilversuch fortgesetzt.
Ziel: vollständige Auflösung des Lipoms.

## Patentansprüche

1. Pharmazeutische Medikation, die einen steroidalen Aromatase-Inaktivator und ein vom steroidalen Aromatase-Inaktivator verschiedenes Antioxidans als Wirkstoffe umfasst, zur Verwendung in einer Prophylaxe oder Therapie einer sexualhormonabhängigen Erkrankung im Menschen, wobei das Antioxidans α-Liponsäure ist, die sexualhormonabhängige Erkrankung Östrogenrezeptor-positiver Brustkrebs ist, und die Medikation topisch appliziert wird, und wobei die Menge des applizierten Antioxidans mindestens 0,5 Gew.-% der Gesamtzusammensetzung beträgt.

2. Pharmazeutische Medikation zur Verwendung gemäß Anspruch 1, wobei die Medikation zur adjuvanten oder neoadjuvanten Therapie von Brustkrebs verwendet wird.

3. Pharmazeutische Medikation zur Verwendung gemäß Anspruch 1, wobei die Medikation zur Primär- oder Sekundärprophylaxe von Brustkrebs verwendet wird.

4. Pharmazeutische Medikation zur Verwendung gemäß irgendeinem der vorangehenden Ansprüche, wobei der steroidale Aromatase-Inaktivator ausgewählt ist aus der Gruppe bestehend aus 4-Hydroxyandrostendion, Exemestane, 4-Acetoxyandrostendion, 5-α-Androst-3-en-17-on und 3-α,4-α-Epoxy-5-α-androstan-17-on.

5. Pharmazeutische Medikation zur Verwendung gemäß irgendeinem der vorangehenden Ansprüche, wobei die Medikation als Wirkstoffe 4-Hydroxyandrostendion und α-Liponsäure umfasst.

6. Pharmazeutische Medikation zur Verwendung gemäß irgendeinem der vorangehenden Ansprüche, wobei der Wirkeingriff allein in einem peripheren, intrakrinen Zielgewebe erfolgt.

## Claims

1. Pharmaceutical medication comprising as active ingredients a steroidal aromatase-inactivator and an antioxidant that is different from the steroidal aromatase-inactivator, for use in prophylaxis or therapy of sex-hormone dependent disease in human, wherein the antioxidant is α-lipoic acid, the sex-hormone dependent disease is estrogen receptor-positive breast cancer, and the medication is applied topically, and wherein the amount of the applied antioxidant is at least 0.5 w/w% of the total composition.

2. Pharmaceutical medication for use according to claim 1, wherein the medication is used in adjuvant or neoadjuvant therapy of breast cancer.

3. Pharmaceutical medication for use according to claim 1, wherein the medication is applied in primary or secondary prophylaxis of breast cancer.

4. Pharmaceutical medication for use according to anyone of the preceding claims, wherein the steroidal aromatase-inactivator is selected from the group consisting of 4-hydroxyandrostenedione, exemestane, 4-acetoxyandrostenedione, 5-α-androst-3-ene-17-one, and 3-α,4-α-epoxy-5-α-androstane-17-one.

5. Pharmaceutical medication for use according to anyone of the preceding claims, wherein the medication comprises as active ingredients 4-hydroxyandrostenedione and α-lipoic acid.

6. Pharmaceutical medication for use according to anyone of the preceding claims, wherein the treatment is effective only in a peripheral, intracrine target tissue.

## Revendications

1. Traitement pharmaceutique, qui comprend comme substances actives un inactivateur stéroïdien de l'aromatase et un antioxydant différent de l'inactivateur stéroïdien de l'aromatase, en vue d'une utilisation dans une prophylaxie ou une thérapie d'une maladie dépendante des hormones sexuelles chez l'être humain,
dans lequel l'antioxydant est l'acide α-lipoïque, la maladie dépendante des hormones sexuelles est un cancer du sein positif pour les récepteurs d'oestrogènes et le traitement est à application topique
et dans lequel la quantité de l'antioxydant appliqué est égale à au moins 0,5 % en poids de la composition totale.

2. Traitement pharmaceutique en vue d'une utilisation selon la revendication 1, dans lequel le traitement est utilisé pour une thérapie adjuvante ou néo-adjuvante du cancer du sein.

3. Traitement pharmaceutique en vue d'une utilisation selon la revendication 1, dans lequel le traitement est utilisé pour la prophylaxie primaire ou secondaire du cancer du sein.

4. Traitement pharmaceutique en vue d'une utilisation selon l'une quelconque des revendications précédentes, dans lequel l'inactivateur stéroïdien de l'aromatase est choisi dans le groupe constitué de : 4-hydroxyandrosténédione, exémestane, 4-acétoxyandrosténédione, 5-α-androst-3-en-17-one et 3-α,4-α-époxy-5-α-androstane-17-one.

5. Traitement pharmaceutique en vue d'une utilisation selon l'une quelconque des revendications précédentes, dans lequel le traitement comprend comme substances actives la 4-hydroxyandrosténédione et l'acide α-lipoïque.

6. Traitement pharmaceutique en vue d'une utilisation selon l'une quelconque des revendications précédentes, dans lequel l'action s'effectue seulement dans un tissu cible périphérique, intracrine.
